(19) Europäisches Patentamt
European Patent Office
Office européen des brevets

(11) **EP 1 685 389 B1**

(12) **FASCICULE DE BREVET EUROPEEN**

(45) Date de publication et mention
de la délivrance du brevet:
**25.04.2007 Bulletin 2007/17**

(51) Int Cl.:
**G01N 22/04** *(2006.01)* **G01N 33/24** *(2006.01)*
**G01R 27/26** *(2006.01)*

(21) Numéro de dépôt: **04805847.3**

(22) Date de dépôt: **19.11.2004**

(86) Numéro de dépôt international:
**PCT/FR2004/050604**

(87) Numéro de publication internationale:
**WO 2005/052561 (09.06.2005 Gazette 2005/23)**

(54) **CAPTEUR ET ENSEMBLE DE MESURES HYDROMETRIQUES**

SENSOR UND GESAMTVORRICHTUNG ZUR HYDROMETRISCHEN MESSUNG

HYDROMETRIC MEASUREMENT PROBE AND ASSEMBLY

(84) Etats contractants désignés:
**AT BE BG CH CY CZ DE DK EE ES FI FR GB GR
HU IE IS IT LI LU MC NL PL PT RO SE SI SK TR**

(30) Priorité: **20.11.2003 FR 0350861**

(43) Date de publication de la demande:
**02.08.2006 Bulletin 2006/31**

(73) Titulaire: **COMMISSARIAT A L'ENERGIE
ATOMIQUE
75015 Paris (FR)**

(72) Inventeur: **HOCLET, Michel
F-91400 ORSAY (FR)**

(74) Mandataire: **Poulin, Gérard
Brevatome
3, rue du Docteur Lancereaux
75008 Paris (FR)**

(56) Documents cités:
**EP-A- 0 460 315** **DE-A- 3 134 662**
**US-A- 4 281 285** **US-A- 5 726 578**

- **CHUDOBIAK W J ET AL: "Recent Advances in Broad-Band VHF and UHF Transmission Line Methods for Moisture Content and Dielectric Constant Measurement" IEEE TRANSACTIONS ON INSTRUMENTATION AND MEASUREMENT, IEEE INC. NEW YORK, US, vol. 28, no. 4, 1 décembre 1979 (1979-12-01), pages 284-289, XP002091380 ISSN: 0018-9456**
- **SOUZA CLAUDINEI F ET AL: "Multi-wire time domain reflectometry (TDR) probe with electrical impedance discontinuities for measuring water content distribution" AGRIC. WATER MANAGE.; AGRICULTURAL WATER MANAGEMENT APR 2 2003, vol. 59, no. 3, 2 avril 2003 (2003-04-02), pages 205-216, XP002279659**

## Description

Un aspect de l'invention décrite ici est un dispositif distribué de mesures hydrométriques formé de moyens électroniques aptes à générer un signal d'excitation en hyperfréquence, d'une ligne de transmission, de cellules de mesure aptes à être connectées en série sur cette ligne de transmission, et de moyens électroniques de traitement des signaux réfléchis. Un autre aspect est le traitement des mesures hydrométriques issues des transducteurs précédents.

Un autre aspect l'invention décrite ici est la séparation entre d'une part les composants électroniques actifs générant le signal d'excitation et traitant les signaux réfléchis, et d'autre part les composants passifs comme la ligne de transmission et les cellules de mesure qui peuvent être placées dans des conditions hostiles, notamment de température ou de rayonnement.

On définit dans ce qui suit la notion de **"câble hyperfréquence"** comme un câble dont les dimensions des conducteurs et les dimensions et caractéristiques du diélectrique entre ces conducteurs sont adéquats pour que ce câble présente une impédance caractéristique de valeur constante dans une large plage de fréquences s'étendant de quelques MHz à plusieurs GHz. Sa structure peut être coaxiale, bifilaire blindée ou bifilaire non blindée. Un tel câble est utilisé pour réaliser la ligne de transmission du dispositif de mesure selon l'invention.

L'hydrométrie désigne ici la mesure du taux d'humidité d'une substance solide. Si le procédé le plus simple pour mesurer ce taux consiste à prélever un échantillon de la matière, à le sécher et mesurer sa perte de masse, il n'est pas toujours réalisable puisqu'il n'est pas toujours possible ou souhaitable de faire de tels prélèvements.

Pour éviter cet inconvénient, on a développé depuis plusieurs années un procédé consistant à envoyer des ondes électromagnétiques dans la matière d'examen, en se fondant sur la grande dépendance de la permittivité en fonction du taux d'humidité de la matière pour des fréquences élevées, la permittivité de l'eau étant très supérieure à celle de corps comme l'argile qu'elle peut imbiber. Les fondements scientifiques de la méthode ont été décrits dans plusieurs publications, sur lesquelles on ne s'étendra pas dans le détail.

Parmi ces procédés, on peut considérer comme proches de l'invention ceux fondés sur la mesure de la permittivité, dans le domaine des fréquences élevées, se rapprochant par valeurs inférieures de la fréquence de relaxation de l'eau, soit 30 GHz à la température ambiante. Ils consistent à envoyer un signal hyperfréquence dans une ligne coaxiale dont le diélectrique (par exemple de l'air) est remplacé au niveau du transducteur par un échantillon de matière (par exemple de l'argile) en équilibre hydrique avec la matière dont on veut mesurer la teneur en eau. Les résultats sont généralement fournis par comparaison avec des tables de résultats théoriques et/ou expérimentaux.

Un problème apparaît pourtant en ce qu'il n'est pas facile de concevoir concrètement un agencement qui permette de répartir entre plusieurs capteurs la puissance transportée par le signal incident afin que chacun de ceux-ci renvoie un signal suffisant pour être analysé, sans prélever une portion excessive du signal total. En outre, même si l'on parvient à répartir l'énergie d'excitation entre différents transducteurs, il est très difficile de limiter les interférences entre ces éventuels transducteurs. Cette limitation est très pénalisante, car de nombreuses applications nécessitent d'effectuer plusieurs mesures simultanées à différents lieux d'un site sans avoir à multiplier le matériel utilisé.

L'article "Recent Advances in Broad-Band VHF and UHF Transmission Line Methods for Moisture Content and Dielectric Constant Measurement" (Walter J. Chudobiak et al., IEEE Trans. on Instrumentation and Measurement, Vol. 28, 1979, Dec., Nr.4) et EP460315 divulguent un dispositif de mesures hydrométriques à hyperfréquence comprenant un câble hyperfréquence le long duquel se trouvent au moins deux postes de mesure.

Pour satisfaire à ces exigences, l'invention concerne un capteur hydrométrique distribué comportant :

- des moyens électroniques aptes à générer des trains d'ondes sinusoïdales à des fréquences prenant plusieurs valeurs en progression arithmétique entre quelques MHz et quelques GHz,

- au moins un câble hyperfréquence, tel que défini précédemment, le long duquel se trouvent au moins deux postes de mesure, chaque poste de mesure ayant d'une part un dispositif séparateur apte à ne prélever de l'onde incidente qu'une partie ayant une énergie suffisante pour que la cellule de mesure renvoie un écho mesurable par les moyens électroniques de lecture, et d'autre part une cellule de mesure proprement dite constituée d'une portion de ligne hyperfréquence dont l'extrémité distale se termine par un court-circuit, cette portion de ligne ayant une paroi externe poreuse ou munie d'orifices, et ayant son diélectrique essentiellement constitué d'un échantillon de matière diélectrique homogène dont la permittivité est une fonction monotone de l'hydrométrie dans le domaine de mesure considéré ;

- des moyens électroniques de lecture permettant, à partir des signaux ayant parcouru le câble hyperfréquence, de numériser ces signaux, de les filtrer en fréquence, de calculer le coefficient de réflexion complexe dans le domaine fréquentiel, d'effectuer une transformée de Fourier pour calculer le coefficient de réflexion complexe dans le domaine temporel, puis de déterminer les valeurs des parties réelles et imaginaires de la permittivité afin de déterminer la mesure de l'humidité et de la température par corrélation à des tables de valeurs expérimentalement établies au préalable à l'aide d'une autre méthode de mesure hydrométrique.

En l'état actuel de la technique, la numérisation des signaux ayant parcouru le câble hyperfréquence ne peut être effectuée directement pour des fréquences allant jusqu'à plusieurs GHz. Il convient alors d'effectuer au préalable une étape de changement de fréquence par des techniques connues de l'homme du métier (multiplication d'une fréquence $F_1$ par une fréquence $F_2$ puis sélection de la bande de fréquences $F_1$ - $F_2$).

Les moyens électroniques aptes à générer des trains d'ondes sinusoïdales à des fréquences prenant plusieurs valeurs en progression arithmétique entre quelques MHz et quelques GHz, doivent être le plus stables possibles. Ils sont de préférence constitués d'un synthétiseur de fréquences stabilisé par quartz. Éventuellement ils peuvent être constitués d'un wobulateur sur lequel nous reviendrons.

Le signal de mesure est appliqué à des voltmètres vectoriels aptes à effectuer le changement de fréquence, un filtrage, la numérisation, un filtrage numérique et la détermination des composantes réelles et imaginaires de la permittivité. Un traitement numérique connu de l'homme du métier peut être ajouté en complément, notamment pour la corrélation avec les tables de mesures préenregistrées.

Un moyen simplifié de réaliser l'excitation et la lecture des signaux consiste à utiliser un analyseur de réseau, comme nous le verrons dans notre description détaillée du fonctionnement. Un tel appareil, bien connu de l'homme du métier, comporte en outre un voltmètre vectoriel $V_R$ constituant une voie de mesures d'une tension de référence en sortie des moyens électronique générant le signal d'excitation. Une telle mesure permet de normer les signaux, c'est-à-dire de les affranchir de paramètres constants dépendants notamment du câble hyperfréquence et des dispositifs de connectique. Enfin, un analyseur de réseau possède des possibilités de calcul numérique.

Le dispositif séparateur apte à ne prélever de l'onde incidente qu'une partie ayant une énergie suffisante est normalement conçu pour ne prélever qu'une énergie juste suffisante pour que la cellule de mesure renvoie un écho mesurable par les moyens électroniques de lecture, soit quelques μw dans l'état actuel de la technologie des appareils de mesure qui seront commentés ultérieurement. Plus généralement, il convient de concevoir ces dispositifs séparateurs de manière à ce que la proportion d'énergie qu'ils dirigent vers la cellule de mesure soit au moins égale à la quantité minimale d'énergie dont cette cellule a besoin.

En réalité, chaque cellule de mesure prélève non pas une quantité constante d'énergie mais une proportion constante. Et la contrainte fonctionnelle à respecter est de veiller à ce que la cellule la plus éloignée de la source reçoive au moins la quantité minimale d'énergie assurant les performances de mesure. Au fur et à mesure que l'onde hyperfréquence parcourt les diverses cellules de mesure, son énergie diminue et il faut modifier la proportion de cette énergie prélevée par chaque séparateur, si l'on souhaite un système de mesures hydrométriques optimisé qui ne prélève de cette onde que le minimum nécessaire.

Or fonctionnellement, si le dispositif de mesure comprend de nombreuses cellules, il va de soi que la première cellule pourra ne prélever qu'un très faible pourcentage de l'énergie incidente, alors que la dernière pourra en prélever la majeure partie. Les caractéristiques dimensionnelles d'une cellule déterminant le pourcentage d'énergie qu'elle prélèvera, une ligne de mesure hydrométrie optimisée devrait comporter des cellules toutes légèrement différentes.

Néanmoins, on peut simplifier la réalisation du capteur hydrométrique distribué en choisissant, de façon sous-optimale, de réaliser des dispositifs séparateurs qui prélèvent de l'onde hyperfréquence une quantité d'énergie supérieure à celle pour laquelle ils devrait normalement être conçu. On peut ainsi obtenir un nombre restreint de variantes dimensionnelles des dispositifs séparateurs utilisés, ou même des dispositifs séparateurs tous identiques en chaque poste de mesure, ce qui abaisse le coût de l'ensemble du capteur hydrométrique distribué.

La réalisation de ce dispositif séparateur peut recourir à tous les moyens connus en hyperfréquences, notamment à des séparateurs de puissance ayant deux sorties très dissymétriques. Dans ce cas, il suffit de connecter à la sortie de plus faible puissance une cellule de mesure simple fonctionnant en cul-de-sac.

Dans les autres cas, qui correspondent à la réalisation préférentielle, ce séparateur qui effectue le prélèvement dissymétrique d'énergie de l'onde hyperfréquence est réalisé par la simple juxtaposition de milieux diélectriques de caractéristiques différentes, et notamment de même nature mais de sections différentes, ceci à impédance caractéristique constante.

Explicitons cela dans le cas où le câble est coaxial. Appelons $d_i$ et de les diamètres intérieur et extérieur du diélectrique du câble hyperfréquence. Le diamètre $d_i$ est aussi le diamètre de l'âme du câble, et le diamètre $d_e$ est aussi le diamètre intérieur du conducteur périphérique de blindage. Appelons $d'_i$ et $d'_e$ les diamètres correspondants du câble rétréci, et $d''_i$ et $d''_e$ les diamètres correspondants pour la cellule de mesure placée autour du câble rétréci. On peut alors exprimer simplement les conditions nécessaires au fonctionnement :

$$d'_i < d_i$$

$$d_i < d'_e < d_e$$

$$d''_i < d_e$$

$$d''_e \geq d_e$$

En outre, la proportion d'énergie entrant dans la cellule de mesure sera fonction de la proportion de surface diélectrique de la cellule (ou s'il y a une rondelle diélectrique la précédant, de cette rondelle diélectrique) en regard de la section en forme de couronne du diélectrique du câble hyperfréquence d'alimentation, c'est-à-dire fonction du rapport :

$$\frac{(\pi/4)\,(\,d_e^2 - d_i^2\,)}{(\pi/4)\,(\,d_e^2 - d''_i{}^2\,)}$$

De manière analogue, la proportion d'énergie entrant dans la partie rétrécie du câble hyperfréquence est fonction du rapport :

$$\frac{(\pi/4)\,(\,d_e^2 - d_i^2\,)}{(\pi/4)\,(\,d'_e{}^2 - d_i^2\,)}$$

Par ailleurs, pour conserver la même impédance caractéristique, préférentiellement fixée à 50 Ohms, le rapport entre les diamètres $d_i$ et de du câble hyperfréquence est le même que le rapport entre les diamètres $d'_i$ et $d'_e$ du câble rétréci dès lors que les diélectriques ont le même indice de permittivité.

Dans le cas où le câble hyperfréquence est bifilaire blindé, la transposition est immédiate à la condition que le diamètre extérieur de l'isolant $d'_e$ du câble rétréci soit plus large que la distance séparant les points les plus éloignés des deux conducteurs dans une section transversale du câble hyperfréquence principal, cette distance pouvant alors jouer le même rôle que $d_i$, bien que les calculs de sections en regard aient alors à être corrigés en conséquence.

Dans le cas où le câble hyperfréquence est bifilaire non blindé, la transposition est immédiate par rapport au cas précédent. En revanche les surfaces métalliques délimitant la cellule de mesure restent parfaitement reliées entre elles tout autour de l'axe du câble, mais ne sont électriquement connectées à rien d'autre.

Afin de bien découpler les deux fonctions suivantes : séparation de l'onde incidente en deux ondes, et interfaçage entre le matériau diélectrique du câble et l'échantillon de matière en équilibre hydrique, il est préférable que chaque cellule de mesure soit précédée, dans le sens de la propagation de l'onde, d'une simple rondelle diélectrique d'adaptation, réalisée en un diélectrique de préférence identique à celui du câble hyperfréquence, et occupant radialement tout l'espace de la cavité sur une certaine longueur mesurée selon l'axe de propagation de l'onde, de l'ordre de 5 à 15 mm. Ensuite l'onde hyperfréquence pénètre dans la cellule de mesure, c'est-à-dire dans une cavité remplie d'un échantillon, homogène et comprimé de façon adéquate, d'une matière en équilibre hydrique avec le milieu extérieur à mesurer. Cette compression adéquate est déterminée expérimentalement.

Dans chacune de ces cellules de mesure, l'onde incidente se propage non plus dans un diélectrique de préférence identique à celui du câble hyperfréquence, mais dans l'échantillon de matière sensible à l'humidité. Les caractéristiques diélectriques de ce matériau doivent varier selon une fonction monotone de l'humidité dans l'étendue de mesure du capteur, et sa fréquence de relaxation doit être supérieure à la fréquence maximale de travail du système de mesure. Il doit en outre être en quantité suffisante pour donner lieu à un signal de réponse d'amplitude suffisante pour les moyens électroniques de lecture, et comprimé de manière adéquate telle que déterminée empiriquement. Le matériau sensible à l'humidité est préférentiellement de l'argile.

Selon le mode de réalisation préférentiel, les moyens de lecture sont situés à la même extrémité du câble hyperfréquence que les moyens de générer des trains d'ondes sinusoïdales, et reliés à ce câble hyperfréquence par un coupleur directif connu de l'homme du métier. Le câble hyperfréquence est alors utilisé en réflexion.

Selon cette première variante, qui utilise le câble hyperfréquence en transmission, il est possible de mesurer à la fois le signal injecté à l'entrée du câble et le signal transmis à l'extrémité distale. Ceci permet aisément de calculer à la fois le coefficient de réflexion et le coefficient de transmission du câble hyperfréquence, d'où un meilleur rapport signal sur bruit des mesures.

Le signal hyperfréquence réfléchi est appliqué à l'entrée d'un premier voltmètre vectoriel $V_A$, tandis que le signal transmis à l'extrémité du câble hyperfréquence est appliqué à l'entrée d'un second voltmètre vectoriel $V_B$. Une résistance adaptée est de préférence connectée aux bornes du voltmètre $V_B$. Il est ainsi possible de mesurer à la fois le coefficient de réflexion complexe et le coefficient de transmission complexe du câble hyperfréquence, dont on déduit par des calculs connus de l'homme du métier la partie réelle et la partie imaginaire de la permittivité. Ces valeurs sont alors corrélées à des mesures d'humidité et de température, effectuées par une autre méthode de mesures lors d'une phase préliminaire d'étalonnage.

Selon une variante plus contraignante, les moyens de lecture sont situés à l'extrémité du câble hyperfréquence opposée à celle reliée aux moyens de générer des trains d'ondes sinusoïdales. Le câble hyperfréquence est alors utilisé en transmission. Dans ce cas en effet on peut ajouter aussi des moyens de mesure du signal électrique hyperfréquence à l'extrémité distale du câble, et en aval les moyens de déterminer le coefficient de transmission complexe de ce câble.

Selon cette seconde variante, qui utilise le câble hyperfréquence en réflexion, un seul voltmètre vectoriel $V_A$ mesure le coefficient de réflexion complexe, dont on déduit par des calculs connus de l'homme du métier la partie réelle et la partie imaginaire de la permittivité. Ces valeurs sont alors corrélées à des mesures d'humidité et de température, effectuées par une autre méthode de mesures lors d'une phase préliminaire d'étalonnage.

Pour ces deux variantes, la réalisation pratique impose qu'un autre voltmètre vectoriel $V_R$ effectue la mesure d'une tension de référence en sortie des moyens électronique générant le signal d'excitation, cette mesure servant à normer les signaux, c'est-à-dire à les affranchir de paramètres constants dépendants notamment du câble hyperfréquence et des dispositifs de connectique.

Il est généralement avantageux en hyperfréquences de s'affranchir des réflexions parasites à l'extrémité distale d'une ligne en plaçant à son extrémité distale une charge adaptée. Il est ici avantageux et naturel de relier l'extrémité distale du câble hyperfréquence à une charge ayant une impédance sensiblement égale à l'impédance caractéristique de ce câble. Mais ce n'est pas impératif en raison du fonctionnement même de l'invention : une extrémité désadaptée, comme par exemple une extrémité « en l'air » (non connectée), se traduit par un signal réfléchi extrêmement fort mais qui arrive après les signaux utiles et peut donc ce fait en être séparé. En revanche, une extrémité désadaptée du point de vue de l'impédance présenterait un risque de saturation de certains circuits du fait de cette amplitude du signal réfléchi.

Pour réaliser l'équilibre hydrique entre l'intérieur de cette cellule, et le milieu extérieur à mesurer, l'invention présente deux variantes. Selon la première variante, la paroi métallique comporte des orifices ou des fentes, de préférence orientées longitudinalement selon l'axe du câble hyperfréquence afin de perturber le moins possible les lignes de courant.

Selon la seconde variante, la paroi externe de cette portion de ligne est poreuse, réalisée par frittage d'un métal inoxydable, comme l'inox, certains bronzes ou le titane.

Les surfaces métalliques conductrices utilisées pour les câbles et la délimitation des cellules de mesure sont préférentiellement en cuivre.

Variantes de séparateurs et de cellules :

Revenons sur le dispositif séparateur apte à ne prélever de l'onde incidente qu'une partie ayant une énergie suffisante, et à la manière de le combiner aux diverses types de câbles hyperfréquence.

La **réalisation préférentielle** d'une cellule de mesure selon l'invention varie très peu selon que le câble hyperfréquence est coaxial ou bifilaire blindé (1er et 2è modes de réalisation) .

Le **premier mode** de réalisation correspond à un câble coaxial, et se caractérise en ce que la cellule de mesure a une structure coaxiale avec le câble hyperfréquence assurant le fonctionnement des cellules de mesure situées en aval, et autour de celui-ci qui présente alors à l'endroit de la cellule un brusque rétrécissement à impédance constante. Elle se caractérise aussi en ce que le dispositif séparateur est réalisé par la simple juxtaposition de milieux diélectriques à l'endroit où le câble hyperfréquence est remplacé par la mise en parallèle de deux milieux : d'une part un diélectrique assurant la continuité du câble, d'autre part la cellule de mesure, ou la rondelle diélectrique d'adaptation s'il y en a une.

On peut encore définir ce premier mode de réalisation en ce que la cellule de mesure forme un cylindre creux délimité par trois surfaces conductrices métalliques en contact : une surface cylindrique intérieure, une surface cylindrique extérieure et un disque plan à l'extrémité distale. Ce cylindre creux est coaxial au câble hyperfréquence et placé autour, celui-ci présentant à cet endroit un brusque rétrécissement du diélectrique et du conducteur extérieur, les dimensions de ces éléments étant néanmoins choisies de manière à ce que l'impédance caractéristique reste la plus constante possible avant, pendant et après le rétrécissement. Il va de soi que, pour maintenir constante l'impédance caractéristique du câble, lorsque le diamètre du diélectrique est brusquement réduit, il faut que le diamètre de l'âme conductrice soit simultanément réduit dans les proportions connues de l'homme du métier.

Le **second mode** de réalisation correspond à un câble hyperfréquence à structure bifilaire blindée. La section de ce câble comporte alors non plus une âme centrale mais deux conducteurs disposés symétriquement par rapport au plan de symétrie de cette section. Ces deux conducteurs sont entourés d'un diélectrique, lui-même entouré d'un blindage conducteur. Au niveau de chaque cellule de mesure, ce diélectrique a un diamètre plus petit, limité par un tronçon de surface cylindrique conductrice qui constitue à la fois le blindage de la ligne ainsi rétrécie et la paroi interne d'une cellule de mesure en forme de cylindre creux, identique à celle décrite dans le cas d'un câble hyperfréquence coaxial. La paroi externe de cette cavité est constituée d'un second tronçon de surface conductrice, poreuse ou comportant des orifices permettant un équilibre hydrique avec le milieu environnant, elle aussi reliée électriquement au blindage du câble hyperfréquence. Cette cavité renferme de préférence une rondelle diélectrique s'étendant radialement entre les deux surfaces conductrices, cette partie étant préférentiellement du même matériau que le câble hyperfréquence, préférentiellement avec une continuité de structure. Le reste de la cavité, jusqu'à le rondelle métallique distale de court-circuit, constitue la cellule de mesure remplie de l'échantillon de matériau en équilibre hydrique avec le milieu à mesurer.

Un **troisième mode** de réalisation d'une cellule de mesure selon l'invention, utilise un câble hyperfréquence de type bifilaire non blindé, c'est-à-dire constitué de deux conducteurs séparés et entourés par un diélectrique pouvant avoir une forme aplatie. La cellule de mesure se situe au moins en partie dans l'épaisseur de ce diélectrique, et revêt une forme analogue aux cellules de mesure décrite précédemment, à ceci près qu'elle n'est électriquement connectée à rien. Si la section du câble hyperfréquence montre un diélectrique dont le contour est extérieur est aplati, par exemple en forme d'ovale, la cellule de mesure peut à son tour être aplatie, par exemple en forme d'ovale.

Un **quatrième mode** de réalisation correspond au cas où le dispositif séparateur n'est plus associé étroitement à le cellule de mesure mais recourt à un dispositif hyperfréquence quelconque, notamment un séparateur de puissance ayant deux sorties très dissymétriques. A titre d'exemple, ce séparateur est un coupleur en T ou en Y dont une des sorties reçoit beaucoup moins de puissance que l'autre. Cette sortie est alors connectée à un tronçon de câble hyperfréquence comparable au câble transmettant l'essentiel de l'onde hyperfréquence, et terminé par une cellule de mesure simplifiée qui ne comporte pas de câble rétréci en son milieu. Le conducteur central est alors non plus une surface conductrice cylindrique mais un simple fil conducteur, de préférence en cuivre, dont l'extrémité distale est connectée à un disque conducteur fermant la cellule.

Dans tous ces cas, la cellule de mesure selon l'invention peut être réalisé d'une grande diversité de manières et de formes, dès lors qu'elle respecte les contraintes ci-dessus, et notamment qu'une portion de ligne en court-circuit voit son diélectrique brusquement remplacé par un échantillon de matière en équilibre hydrique avec le milieu à mesurer. Elle est conçue pour avoir une impédance peu différente de celle du câble hyperfréquence qui l'alimente. Si la cellule, comme c'est généralement le cas, a un diamètre supérieur à celui du câble de dérivation, l'onde hyperfréquence circulant dans le diélectrique du câble doit être amenée dans cette zone de plus grand diamètre par une pièce diélectrique intermédiaire avant d'entrer directement en contact avec l'échantillon de matière en équilibre hydrique.

Fonctionnement :

Il est maintenant possible de comprendre le fonctionnement du dispositif séparateur apte à ne prélever qu'une partie de l'onde hyperfréquence incidente lorsqu'il est étroitement associé à la cellule de mesure comme dans les trois premiers modes de réalisation. L'onde incidente circulant dans le diélectrique du câble hyperfréquence voit, en arrivant au poste de mesure, une zone centrale pourvue d'un diélectrique comparable à celui du câble, une zone périphérique constituée de la cellule de mesure proprement dite, ces deux zones étant séparées par une surface conductrice reliée électriquement au conducteur périphérique du câble hyperfréquence, et l'énergie se répartissant entre ces deux zones selon leurs sections respectives.

Les sections respectives des diélectriques correspondant à ces deux milieux sont choisies en fonction de la proportion de l'énergie incidente que l'on veut conserver dans la partie aval du câble hyperfréquence, et donc du nombre de transducteurs en aval. Ce choix doit toutefois assurer à chaque cellule de mesure une énergie minimale pour que le signal en retour puisse être lu avec un rapport signal sur bruit suffisant. Il y a donc lieu de déterminer, pour l'installation d'un capteur hydrométrique distribué selon l'invention, le rapport que l'on juge optimal entre le nombre de postes de mesure et la précision du signal.

Indépendamment du mode de réalisation des cellules de mesure, **l'excitation** du câble hyperfréquence connecté aux cellules de mesure peut se faire de plusieurs manières. Chaque manière doit soumettre ce câble et les cellules de mesure à une pluralité de signaux recouvrant une pluralité de fréquences, proches les unes des autres, l'ensemble couvrant une bande de fréquences allant de quelques MHz à plusieurs GHz.

La manière la plus simple consiste à générer le signal hyperfréquence d'excitation, par un synthétiseur de fréquences très stable, piloté par quartz. Il est aussi possible d'utiliser un wobulateur, c'est-à-dire un générateur d'onde sinusoïdale sur une petite échelle de temps, mais dont la fréquence varie continûment d'une valeur minimale à une valeur maximale, ou inversement. Néanmoins une telle méthode ne permet pas d'effectuer sur le signal de lecture un filtrage fréquentiel sur une bande aussi étroite que lorsqu'on recourt à un synthétiseur de fréquences. Le rapport signal sur bruit du dispositif de mesure s'en trouve pénalisé.

Préférentiellement, afin d'améliorer le rapport signal sur bruit, on choisit d'appliquer une même fréquence sinusoïdale pendant un temps suffisant pour l'établissement d'un régime d'équilibre, puis de choisir une nouvelle fréquence et de répéter l'opération, et ainsi de suite. Ainsi, à chaque instant l'excitation s'effectue sur une bande de fréquence extrêmement étroite, ce qui permet un filtrage plus efficace du signal reçu. Ce filtrage est effectué à plusieurs reprises tout au long de la chaîne hyperfréquence, comme il est pratiqué habituellement dans cette technique. Le filtrage le plus étroit est effectué numériquement, juste après la numérisation. Pour obtenir de bonnes performances, il est à bande étroite : quelques dizaines de Hz, voir quelques Hz.

Enfin, une troisième méthode plus adaptée aux laboratoires consisterait à générer des impulsions d'excitation aussi proches que possible d'impulsions de Dirac, aptes à mesurer la réponse fréquentielle du système de mesure. Elle ne sera pas développée ici car d'une part elle est connue de l'homme du métier et d'autre part elle convient mal aux applications industrielles.

La manière préférentielle consistant à appliquer une même fréquence pendant un temps suffisant, puis à changer cette fréquence, peut être décrite plus en détail comme suit.

Les fréquences d'excitation successives $f_i$ sont choisies de manière à former une progression arithmétique lorsque i varie, ceci pour permettre le calcul de la transformée de Fourier. Selon notre réalisation, 1601 points de mesure sont placés en progression arithmétique entre une fréquence minimale de 3 MHz et la fréquence maximale de 6 GHz.

Le temps entre deux trains de sinusoïdes successifs doit être suffisamment long pour permettre l'établissement d'un régime permanent dans lequel coexistent le signal d'excitation et l'écho renvoyé par chaque cellule de mesure. La distinction de chaque écho permet de localiser la cellule qui l'a engendrée. À titre indicatif dans notre réalisation préférentielle décrite ultérieurement, ce temps entre deux trains de sinusoïdes successifs est de 187,5 ms.

Lorsque chacune des cellules de mesure du système est soumise à ces ondes d'excitation, elle est le siège d'oscillations amorties entre le fond et l'entrée de cette cellule, chaque réflexion sur l'entrée de la cavité donnant lieu à l'émission d'une oscillation qui retourne vers la source.

La transformée de Fourier de cette réponse, permettant de passer des fréquences aux temps, est aisée à interpréter. Chaque cellule de mesure génère ainsi un signal réfléchi représenté en fonction du temps par une succession de pics équidistants, d'amplitudes décroissantes constituant sa signature. Cette distance constante, permet de déterminer la partie réelle de la permittivité $\varepsilon(\omega)$ de la cellule, qui est liée au taux d'humidité.

En théorie, si cette cellule a une longueur « 1 » selon la direction du vecteur d'onde, le chemin que doit parcourir cette onde hyperfréquence dans la cellule est $n_{21}$, où n est l'indice de réfraction de l'échantillon de matériau. Si le coefficient d'extinction ou d'amortissement $\chi$ n'est pas négligeable, comme lorsqu'on s'approche de la fréquence de transition de l'eau (approximativement vers 4 à 5 GHz et au-dessus), les composantes réelle $\varepsilon'_r$ et imaginaire $\varepsilon''_r$ recherchées de la permittivité $\varepsilon(\omega)$ sont données par :

$$\mathrm{n} + \mathrm{j}\ \chi = \sqrt{\varepsilon(\omega)} = \sqrt{(\varepsilon'_r + j\varepsilon''_r)}.$$

En pratique, lorsque la fréquence maximale de travail est loin de la fréquence de relaxation de l'eau (approximativement en dessous de 1 GHz), il suffit de considérer que l'indice de réfraction pour un tronçon de ligne ou une cellule de longueur 1 est lié à la composantes réelle $\varepsilon'_r$ par :

$$\mathrm{n}_1 = \sqrt{\varepsilon'_{rl}}.$$

La relation théorique montre le lien entre le coefficient d'amortissement des raies et la partie imaginaire $\varepsilon''_r$ de la permittivité $\varepsilon(\omega)$, elle-même liée à la température.

Les grandeurs caractérisant le matériau à mesurer sont l'humidité et la température. Les grandeurs caractérisant le signal du capteur sont la distance entre les raies observables dans la représentation temporelle du signal ayant parcouru le câble, qui est principalement liée à la permittivité réelle, et les amplitudes relatives de chaque raie du signal d'un capteur, qui sont principalement liées à la conductivité du matériau sensible et donc a sa permittivité imaginaire. L'étalonnage complet d'un capteur doit prendre en compte ces quatre grandeurs, c'est-à-dire déterminer à partir des deux caractéristiques du signal les deux caractéristiques du matériau.

Les portions de ligne séparant deux transducteurs successifs pourraient a priori générer, avec les échos portant l'information utile, des produits d'intermodulation parasites ; en pratique, l'invention limitant considérablement l'amplitude de l'écho portant l'information utile (par rapport à une cavité résonnante de l'art antérieur), ces produits d'intermodulation sont si faibles qu'ils se confondent avec le bruit de fond.

La **lecture** des valeurs d'hydrométrie et de température ne peut avoir lieu qu'après une phase préalable d'étalonnage, qui s'effectue à l'aide d'une autre méthode de mesure comme le séchage d'échantillons déjà mentionné. La lecture proprement dite des valeurs d'hydrométrie et de température s'effectue expérimentalement par corrélation à des tables de résultats obtenues lors de la phase d'étalonnage.

Ces mesures sont reliées à la teneur en eau et à la température par les équations connues de l'électromagnétisme. On peut aussi utiliser un voltmètre vectoriel ou un analyseur de réseau comme par exemple le HP8753B de Hewlett Packard, qui donne directement la partie réelle et la partie imaginaire du signal reçu. Pour plus de précision, on peut se référer à la note d'application Hewlett-Packard, Test and measurement application 95-1 dénommée : « S-parameters techniques », chapitre 6 : « Measurement of S-parameters ».

Toutefois, ces calculs étant longs, la lecture des mesures de teneur en eau et de température s'effectue préférentiellement de manière expérimentale par corrélation à des tables de résultats obtenues au préalable, dans une phase d'étalonnage, à l'aide d'une autre méthode de mesure comme le séchage d'échantillon déjà mentionné.

L'étalonnage varie avec les caractéristiques de chaque cellule de mesure, notamment les dimensions du diélectrique dans la partie où il est rétréci et les dimensions de la cavité transductrice, ainsi que la nature du diélectrique. L'étalonnage effectué sur une cellule de mesure reste donc utilisable pour une autre cellule de mesure ayant en commun ces caractéristiques.

Lorsque plusieurs cavités hyperfréquence sont ainsi placées en divers postes de mesure, en série sur un câble hyperfréquence, chacun d'eux renvoie sa propre signature mais avec un décalage temporel dépendant de sa distance à la source d'excitation. Il est alors aisé de distinguer la réponse de chaque cellule de mesure.

La portion de câble hyperfréquence située entre deux transducteurs successifs se comporte elle aussi comme une portion de ligne hyperfréquence dont l'extrémité est la discontinuité introduite par la cellule de mesure suivante. Elle présente donc à son tour, même si c'est de façon moins marquée, un fonctionnement en cavité hyperfréquence. Mais la faiblesse de l'énergie prélevée par chaque transducteur réduit en pratique l'amplitude de tels échos à une valeur négligeable.

L'invention sera maintenant **décrite en liaison aux figures.**

La figure 1 schématise une réalisation préférentielle d'un dispositif transducteur selon l'invention, composé d'un séparateur et d'une cellule de mesure associés, où cette cellule de mesure est de type coaxial, montée sur un câble coaxial.

La figure 2 schématise une deuxième réalisation une réalisation préférentielle d'un dispositif transducteur selon l'invention, composé d'un séparateur et d'une cellule de mesure associés, utilisant un câble bifilaire blindé.

La figure 3 schématise une autre réalisation d'un dispositif transducteur selon l'invention, composé d'un séparateur et d'une cellule de mesure associés utilisant un câble bifilaire non blindé.

La figure 4 schématise une troisième réalisation d'une cellule de mesure simplifiée selon l'invention, c'est-à-dire dont le séparateur de puissance est constitué par **un répartiteur de puissance en Y**, dont une sortie est une cellule de mesure et l'autre sortie la partie aval de la ligne coaxiale.

La figure 5 schématise un dispositif distribué de mesures hydrométrique conforme à l'invention.

Description détaillée de la réalisation préférentielle :

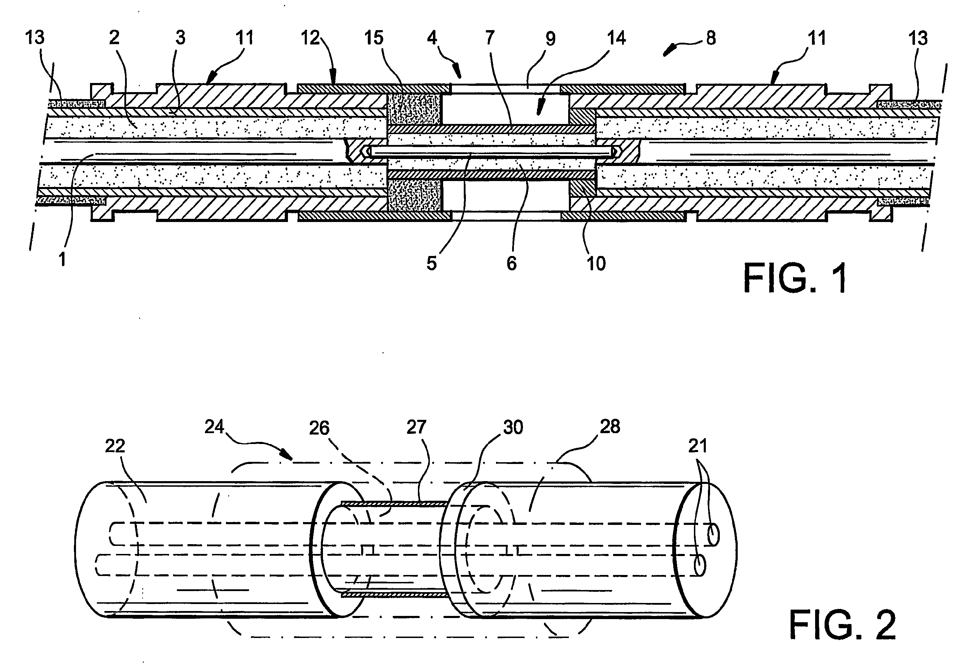

La **figure 1** schématise une réalisation d'un dispositif transducteur composé d'un séparateur et d'une cellule de mesure associés, où cette cellule de mesure est de type coaxial, montée sur un câble coaxial. Ce câble comprend au centre une âme 1 conductrice de l'électricité, de diamètre $d_i$, qu'enrobe une gaine diélectrique 2 en Téflon et un conducteur périphérique 3 constitué ici d'un tube de cuivre de diamètre intérieur de , mais qui pourrait dans d'autres réalisations être une tresse de cuivre. Il est entouré d'une gaine isolante et protectrice 13.

Conformément à l'invention, ce câble coaxial est brusquement rétréci au niveau de la cellule de mesure 14 qui on mesure l'humidité du matériau environnant dans lequel le câble est enterré, comme de l'argile de confinement de déchets de combustible nucléaire. Dans ce cas la distance entre deux cellules de mesure consécutives est d'environ un mètre. Pour une réalisation expérimentale, il a été choisi de réaliser des associations d'une cellule de mesure et d'un séparateur en tant qu'éléments distincts du câble hyperfréquence, reliées à lui de part et d'autre par des connecteurs coaxiaux miniatures de type SMA. Le tronçon de câble hyperfréquence rétréci comporte une âme 5 de diamètre extérieur $d'_i$, qui s'enfonce dans les orifices centraux des connecteurs placés de part et d'autre, un diélectrique 6 lui aussi en Téflon, et un conducteur tubulaire 7 fait d'un morceau de tube de cuivre, d'un diamètre intérieur $d'_e$ de l'ordre de 2,4 mm, inférieur au diamètre intérieur de du conducteur périphérique 3 du câble hyperfréquence situé de part et d'autre. Cette dernière contrainte est nécessaire pour que l'énergie circulant dans le diélectrique du câble hyperfréquence puisse se répartir entre la partie aval de ce même câble et la cellule de mesure. Les isolants des câbles ainsi étant du Téflon pour conserver la même impédance caractéristique, fixée à 50 Ohms, le rapport entre les diamètres $d_i$ et de du câble hyperfréquence est le même que le rapport entre les diamètres $d'_i$ et $d'_e$ du câble rétréci.

La proportion d'énergie de l'onde hyperfréquence envoyée par le séparateur vers la cellule de mesure 14 ou la rondelle diélectrique d'adaptation 15 qui la précède est déterminée par la surface commune entre la section droite du diélectrique 2 et la section transversale gauche de la cellule 14 ou de la rondelle diélectrique d'adaptation 15. Elle est déterminée de manière à ce que l'écho émis en retour vers les moyens de lecture atteigne ceux-ci avec une énergie de l'ordre d'un à 2 $\mu$W.

Les éléments coaxiaux 11 qui assurent la continuité électrique sont des éléments de connecteurs coaxiaux du commerce dont on n'a pas détaillé la représentation du presse-étoupe.

Avec cette disposition, l'onde incidente d'excitation émise à gauche du câble coaxial et courant dans le diélectrique 2, en parvenant au poste de mesure 4, traverse presque totalement celui-ci en passant par la portion de câble hyperfréquence rétrécie 5, 6, 7 ; mais une très faible partie de l'énergie de l'onde incidente , de l'ordre d'1 à 2 $\mu$w, est transmise hors de la section d'enveloppe 7 et passe donc dans la rondelle diélectrique d'adaptation 15, puis la cellule de mesure 14 remplie par un échantillon d'argile.

L'anneau 10, conducteur de l'électricité, établit un court-circuit entre le conducteur tubulaire 7 et le conducteur tubulaires extérieur 12 à l'extrémité distale de la cellule de mesure 14, de manière à la faire fonctionner en cavité résonante hyperfréquence. La figure 1 montre que ces éléments 10 et 12 sont en contact électrique via le corps du connecteur coaxial 11. Il va de soi que ces deux pièces ne forment pour la cellule de mesure 14, qu'une seule surface plane conductrice s'étendant radialement. La portion des ondes arrivant dans cette cavité se réfléchit sur le court circuit distal, revient vers la jonction entre le diélectrique 2 et la cellule de mesure 14. Là, une faible partie de cette onde traverse cette jonction et retourne vers l'entré du câble où elle est analysée, alors que la majeure partie de cette onde se réfléchit et repart dans la cavité où elle se réfléchit à nouveau sur le court-circuit distal. Et ainsi de suite jusqu'à l'amortissement de cette onde.

La douille de raccordement 8 peut directement jouer le rôle de partie mâle du connecteur coaxial miniature dès lors qu'elle présente un état de surface satisfaisant et que l'âme 5 a le diamètre de la broche de connexion correspondante.

La portion de l'onde incidente circulant dans le diélectrique 2 et transmise à la cellule de mesure 14 est déterminée par la surface commune entre la section transversale droite du diélectrique 2 et la section transversale gauche de la cavité. Elle peut donc être ajustée en fonction des diamètres des câbles coaxiaux utilisés.

Les discontinuités de structures diélectriques à travers les cellules de mesure 14, produisent une déperdition du signal qui peut être importante si le dressage des faces en regard est imparfait. Il est donc préconisé que les diélectriques soient fabriqués non seulement à partir du même matériau, mais si possible avec une continuité de structure.

La plus grande partie du signal a cependant franchi la cellule de mesure 14 en passant par le diélectrique 6 ; elle atteint ultérieurement un autre poste de mesure 4, situé plus loin sur le câble et semblable à celui qui est illustré, et des phénomènes analogues s'y produisent. On peut ainsi réaliser aisément une ligne de plus d'une dizaine de cellules de mesure, la limite dépendant principalement des performances du dispositif de lecture. Si on souhaite optimiser les performances d'un tel système de mesure comportant un grand nombre de cellules, il est préférable de modifier progressivement la proportion de l'énergie incidente envoyée dans la cellule de mesure, comme exposé précédemment.

Pour simplifier la mise en oeuvre et profiter des possibilités de traitement, nous avons utilisé comme moyen de réaliser l'excitation et la lecture des signaux un analyseur de réseau Hewlett Packard de type HP 8510. Un tel appareil comporte un synthétiseur de fréquences utilisé pour générer les signaux d'excitation. Il comporte aussi trois voltmètres vectoriels $V_A$, $V_B$ et $V_R$, le troisième constituant une voie de mesures d'une tension de référence prise en dérivation au début du câble hyperfréquence à l'aide d'un coupleur de puissance hyperfréquence. Les autres voies de mesure sont couplées au câble de la même façon, la voie A à l'origine du câble hyperfréquence, et la voie B, utilisée seulement lors des expérimentations en transmission, à l'extrémité distale du câble.

Plusieurs filtrages internes à l'appareil optimisent les mesures. Après numérisation, un changement de fréquence du signal utile est effectué avec une fréquence F2 qui diffère de la fréquence initiale F1 de 10 kHz. Plusieurs autres réglages ont été essayés, y compris avec des bandes passantes de quelques Hz.

L'extrémité de la ligne, fermée sur une charge adaptée, ne renvoie pas d'écho. Mais des essais ont aussi été effectués avec des charges désadaptées.

L'analyseur de réseau peut donc mesurer le coefficient de réflexion des ondes $S_{11}(\omega)$ à l'entrée de la ligne par sa composante réelle et sa composante imaginaire qui permettent de calculer pour chaque cellule de mesure les valeurs de permittivité réelle et imaginaire $\varepsilon(\omega)$ et $\mu_r(\omega)$.

Le rapport signal sur bruit des mesures dépend du rapport signal sur bruit de l'analyseur, qui est de $10^5$. Toutefois en pratique les inhomogénéités du câble ramènent un bruit de fond constant qui diminue le rapport signal sur bruit à une valeur effective de $10^4$. En outre, quand le signal à mesurer devient trop faible vis-à-vis des caractéristiques d'entrée de l'analyseur, le rapport signal sur bruit chute.

La **figure 2,** schématise une variante de réalisation d'un dispositif transducteur selon l'invention, composé d'un séparateur et d'une cellule de mesure associés utilisant un câble bifilaire non blindé, où le câble hyperfréquence est réalisé en câble bifilaire blindé, et où la cellule de mesure conserve sa structure coaxiale. L'âme est simplement remplacée par deux conducteurs assez proches l'un de l'autre relativement au diamètre extérieur du diélectrique.

Cette cellule de mesure est alors composée à partir du câble hyperfréquence comprenant une paire d'âmes conductrices identiques 21 et un diélectrique 22 dont la périphérie est retirée par usinage au niveau de la cellule de mesure 24 pour ne laisser subsister qu'une section réduite de diélectrique 26. On dispose ensuite une section d'enveloppe conductrice 27 autour de la section de diélectrique 26, comme précédemment, puis un anneau conducteur 30 à l'extrémité distale de la cavité ainsi formée, avant de disposer une douille de raccordement 28 semblable à la douille 8 déjà rencontrée autour du câble à l'endroit des cellules de mesure selon le mode préférentiel de réalisation. Il est manifeste que les phénomènes électromagnétiques rencontrés sont les mêmes.

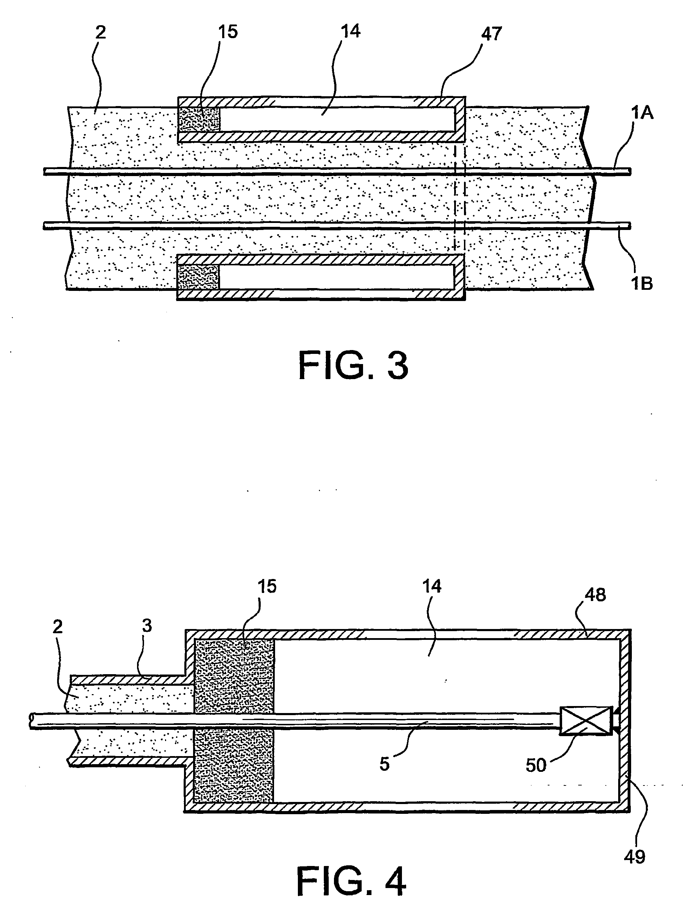

La **figure 3,** schématise une variante de réalisation d'un dispositif transducteur selon l'invention, composé d'un séparateur et d'une cellule de mesure associés utilisant un câble bifilaire non blindé, où la cellule de mesure conserve sa structure coaxiale. Par rapport à la variante qui précède, la seule modification est l'absence de connexion électrique entre les surfaces qui délimitent la cellule de mesure et les deux conducteurs du câble hyperfréquence. La cellule de mesure 14 est délimitée par une enveloppe métallique 47 et a une forme annulaire. L'âme conductrice comprend ici deux éléments parallèles 1A et 1B, qui traversent le creux formé au milieu de l'enveloppe métallique 47. L'enveloppe métallique 47 et la rondelle 15 forment une cavité close pour la cellule 14.

La figure 4 une troisième réalisation d'une cellule de mesure simplifiée selon l'invention, c'est-à-dire dont le séparateur de puissance est constitué par un répartiteur de puissance en Y, dont une sortie est une cellule de mesure et l'autre sortie la partie aval de la ligne coaxiale. Elle comprend une résistance 50 d'adaptation à l'impédance du câble placée sur l'âme 5 au bout de la cellule de mesure 14. L'âme 5 pénètre dans la cellule de mesure 14 et elle est soudée au fond 49 de l'enveloppe métallique 48.

Les réalisations décrites précédemment utilisent des connecteurs et des moyens de mesure de laboratoire hyperfréquences qui sont coûteux. Pour une utilisation industrielle, ces moyens pourraient être remplacés par des moyens moins coûteux et remplissant les mêmes fonctions, comme l'association à des voltmètres vectoriels d'un pont réflectrométrique hyperfréquence et à des coupleurs directifs. Mais il est préférable d'utiliser les enseignements ci-dessus pour concevoir une installation spécifique telle que schématisée ci-après.

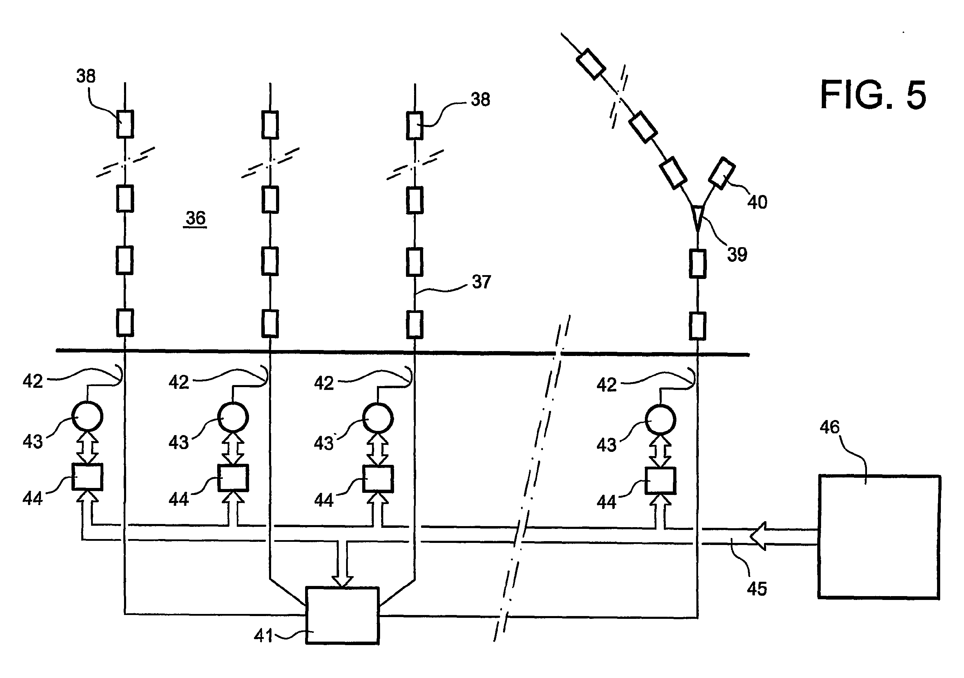

La figure 5 illustre une réalisation complète de l'invention. Une barrière ouvragée 35 confine un volume d'argile de l'extérieur. Une série de câbles coaxiaux 37 y pénètre, et ces câbles coaxiaux 37, munis de transducteurs 38 formés d'un séparateur et d'une cellule de mesure associée semblables à ceux qu'on a décrits et connectées en cascade. Un séparateur de puissance dissymétrique réalisé par un coupleur de puissance 39 permet de connecter à l'une des lignes une cellule de mesure simplifiée 40. Chacun des câbles coaxiaux 37 est alimenté par un coffret de multiplexage 41 hors du volume d'argile 36, à un synthétiseur de fréquences piloté par quartz et stabilisé par PLL, qui génère des trains d'ondes sinusoïdales à des fréquences prenant plusieurs valeurs en progression arithmétique entre quelques MHz et quelques GHz. Les signaux ayant parcouru chaque câble (ici en réflexion) sont prélevés par des coupleurs directionnels 42 et appliqués à l'entrée du voltmètre vectoriel 43 dédié à ce câble. Un dispositif de traitement 42 à microcontrôleur est associé à chacun de ces voltmètres vectoriels, qui sont connectés par un bus bidirectionnel 45 à un système informatique qui permet de déterminer les paramètres de fonctionnement et reçoit des valeurs numériques des parties réelles et imaginaires de la permittivité dans chacune des cellules de mesure, avant d'effectuer la comparaison avec les tables de mesures préenregistrées. L'ensemble constitue un exemple de dispositif de mesures hydrométriques distribuées conforme à l'invention.

L'invention s'applique à tous les cas où une mesure distribuée d'hydrométrie est nécessaire sur un même câble ou un ensemble de câbles connectés en parallèle. Le coût élevé de l'analyseur de réseau peut être diminué par la réalisation d'une électronique dédiée. En outre ce coût est associé à un nombre important de postes de mesure.

Les constituants rendent l'invention intrinsèquement résistante aux rayonnements ionisants, ce qui la rend particulièrement utile pour la mesure de la teneur en eau des argiles entourant des conteneurs de déchets nucléaires.

**Revendications**

1. Dispositif de mesures hydrométriques à hyperfréquence comprenant :

   - des moyens électroniques aptes à générer des trains d'ondes sinusoïdales à des fréquences prenant plusieurs valeurs en progression arithmétique entre quelques MHz et quelques GHz,
   - au moins un câble hyperfréquence le long duquel se trouvent au moins deux postes de mesure (4), chaque poste de mesure ayant d'une part un dispositif séparateur apte à ne prélever de l'onde incidente qu'une partie ayant une énergie suffisante pour que la cellule de mesure renvoie un écho mesurable par les moyens électroniques de lecture, et d'autre part une cellule de mesure (14) constituée d'une portion de ligne hyperfréquence dont l'extrémité distale se termine par un court circuit, cette portion de ligne ayant une paroi externe poreuse ou munie d'orifices, et ayant son diélectrique essentiellement constitué d'un échantillon de matière diélectrique homogène dont la permittivité est une fonction monotone de l'hydrométrie dans le domaine de mesure considéré,
   - des moyens électroniques de lecture permettant, à partir des signaux ayant parcouru le câble hyperfréquence, de déterminer les valeurs des parties réelles et imaginaires de la permittivité afin de déterminer la mesure de l'humidité et de la température par corrélation à des tables de valeurs expérimentalement établies au préalable à l'aide d'une autre méthode de mesure hydrométrique.

2. Dispositif de mesures hydrométriques, conforme à la revendication 1, dans lequel les moyens électroniques de lecture comportent des moyens : de numériser ces signaux, de les filtrer en fréquence, de calculer le coefficient de réflexion complexe dans le domaine fréquentiel, d'effectuer une transformée de Fourier pour calculer le coefficient de réflexion complexe dans le domaine temporel, puis de déterminer les valeurs des parties réelles et imaginaires de la permittivité.

3. Dispositif de mesures hydrométriques, conforme à l'une quelconque des revendications 1 ou 2, dans lequel les moyens de lecture sont situés à la même extrémité du câble hyperfréquence que les moyens de générer des trains d'ondes sinusoïdales, et sont reliés à ce câble hyperfréquence par un coupleur directif.

4. Dispositif de mesures hydrométriques, conforme à l'une quelconque des revendications 1 à 3, dans lequel le câble hyperfréquence est coaxial.

**5.** Dispositif de mesures hydrométriques, conforme à l'une quelconque des revendications 1, 2, ou 3, dans lequel le câble hyperfréquence est bifilaire blindé.

**6.** Dispositif de mesures hydrométriques, conforme à l'une quelconque des revendications 1 à 3, dans lequel le câble hyperfréquence est bifilaire non blindé.

**7.** Dispositif de mesures hydrométriques, conforme à l'une quelconque des revendications 1 à 6, dans lequel la cellule de mesure est coaxiale avec le câble hyperfréquence, et celui-ci présente un brusque rétrécissement au niveau de cette cellule.

**8.** Dispositif de mesures hydrométriques conforme à la revendication 1 et à l'une quelconque des revendications 3 à 5, dans lequel le dispositif apte à ne prélever de l'onde incidente qu'une partie ayant une énergie suffisante est un répartiteur de puissance, et la cellule de mesure est placée en dérivation par rapport au câble hyperfréquence.

**9.** Dispositif de mesures hydrométriques conforme à la revendication 1 dans lequel la paroi externe de la cellule de mesure est pourvue de fentes orientées selon le vecteur de propagation de l'onde.

**10.** Dispositif de mesures hydrométriques conforme à la revendication 1 dans lequel la paroi externe de la cellule de mesure est poreuse.

**11.** Dispositif de mesures hydrométriques conforme aux revendications 1, 3 et 6 ou aux revendications 1, 4 et 6, dans lequel la cellule de mesure comporte une cavité de forme cylindrique creuse délimitée par :

- une surface intérieure cylindrique conductrice, constituant également le blindage de la partie rétrécie du câble hyperfréquence,
- une surface extérieure cylindrique conductrice, électriquement connectée par ses deux extrémités au blindage des deux tronçons de câble hyperfréquence qui l'entourent,
- la partie distale de cette cavité étant constituée d'une rondelle conductrice mettant en contact sur 360° les deux surfaces cylindriques et la partie aval du câble hyperfréquence,

cette cavité étant remplie à son extrémité tournée vers le générateur par un diélectrique identique à celui du câble et occupant tout l'espace entre les deux cylindres sur une longueur de quelques millimètres, et étant remplie dans la partie restante de l'échantillon de matière diélectrique homogène dont la permittivité est une fonction monotone de l'hydrométrie.

**12.** Dispositif de mesures hydrométriques conforme aux revendications 1, 5 et 6, dans lequel la cellule de mesure comporte une cavité de forme cylindrique creuse délimitée par :

- une surface intérieure cylindrique conductrice, de diamètre inférieur au diamètre le plus petit du diélectrique entourant les deux conducteurs,
- une surface extérieure cylindrique conductrice,
- la partie distale de cette cavité étant constituée d'une rondelle conductrice mettant en contact sur 360° les deux surfaces cylindriques,

cette cavité étant remplie à son extrémité tournée vers le générateur par un diélectrique identique à celui du câble et occupant tout l'espace entre les deux cylindres sur une longueur de quelques millimètres, et étant rempli dans la partie restante de l'échantillon de matière diélectrique homogène dont la permittivité est une fonction monotone de l'hydrométrie.

**13.** Dispositif de mesures hydrométriques conforme à l'une quelconque des revendications 1 à 12, **caractérisée en ce qu'**une ou plusieurs cellules de mesure distale(S) prélèvent une plus grande proportion de l'onde hyperfréquence incidente que les cellules de les plus proches de la source.

**14.** Dispositif de mesures hydrométriques conforme à l'une quelconque des revendications 1 à 12, **caractérisée en ce que** les diélectriques du câble hyperfréquence et de la cellule de mesure ont une structure continue.

**15.** Dispositif de mesures hydrométriques conforme à l'une quelconque des revendications 1 à 13, comportant un même générateur de trains d'ondes sinusoïdales, un dispositif de multiplexage commutant ces trains d'ondes successivement à une extrémité de plusieurs câbles hyperfréquence, un voltmètre vectoriel (43) connecté à chacun de ces câbles hyperfréquence et les moyens électroniques permettant de calculer le coefficient de réflexion complexe dans le domaine fréquentiel, d'effectuer une transformée de Fourier pour calculer le coefficient de réflexion complexe dans le domaine temporel, puis de déterminer les valeurs des parties réelles et imaginaires de la permittivité afin de déterminer la mesure de l'humidité et de la température par corrélation à des tables de valeurs expérimentalement établies au préalable à l'aide d'une autre méthode de mesure hydrométrique.

**16.** Dispositif de mesures hydrométriques conforme à la revendication 1 dans lequel les moyens de lecture sont situés à l'extrémité du câble hyperfréquence opposée à celle reliée aux moyens de générer des trains d'ondes sinusoïdales.

**17.** Ensemble de mesure hydrométrique comprenant au moins un capteur selon l'une quelconque des revendications 1 à 16, **caractérisé en ce que** le générateur de trains d'ondes sinusoïdaux et les moyens électroniques de lecture sont constitués par un analyseur de réseau.

**18.** Ensemble de mesure hydrométrique comprenant au moins un capteur selon l'une quelconque des revendications 1 à 14, **caractérisé en ce que** le générateur de trains d'ondes sinusoïdaux est un synthétiseur de fréquences, les moyens électroniques de lecture sont constitués par un voltmètre vectoriel (43) associé à des moyens de traitement numériques.

**Claims**

**1.** A device for ultra-high frequency hydrometric measurements comprising:

- electric means capable of generating sine wave trains at frequencies assuming several values in arithmetic progression between a few MHz and a few GHz,
- at least one ultra-high frequency cable along which at least two measuring stations (4) are found, each measuring station having a separator device capable of only sampling from the incident wave a portion with sufficient energy so that the measuring cell sends back an echo measurable by electronic read-out means, on the one hand, and a measuring cell (14) consisting of a ultra-high frequency line portion, on the other hand, the distal end of which is terminated by a short circuit, this line portion having an external wall either porous or provided with ports, and having its dielectric essentially consisting of a sample of homogeneous dielectric material for which permittivity is a monotonous function of the hydrometry in the relevant measurement domain,
- electronic read-out means with which, from signals having traveled through the ultra-high frequency cable, values of the real and imaginary parts of the permittivity may be determined, in order to determine the measurement of humidity and temperature by correlation with tables of values experimentally established beforehand by means of another hydrometric measurement method.

**2.** A device for hydrometric measurements, according to claim 1, wherein the electronic read-out means include means: for digitizing these signals, for filtering them in frequency, for calculating the complex reflection coefficient in the frequency domain, for performing a Fourier transform in order to calculate the complex reflection coefficient in the time domain, and then for determining the values of the real and imaginary parts of the permittivity.

**3.** The device for hydrometric measurements, according to any of claims 1 or 2, wherein the read-out means are located at the same end of the ultra-high frequency cable as the means for generating sine wave trains, and are connected to this ultra-high frequency cable by a directive coupler.

**4.** The device for hydrometric measurements, according to any of claims 1 or 3, wherein the ultra-high frequency cable is coaxial.

**5.** The device for hydrometric measurements, according to any of claims 1, 2 or 3, wherein the ultra-high frequency cable is shielded and bifilar.

**6.** The device for hydrometric measurements, according to any of claims 1 to 3, wherein the ultra-high frequency cable is unshielded and bifilar.

**7.** The device for hydrometric measurements, according to any of claims 1 to 6, wherein the measuring cell is coaxial with the ultra-high frequency cable, and the latter has sudden narrowing at this cell.

**8.** The device for hydrometric measurements, according to claim 1 and to any of claims 3 to 5, wherein the device capable of only sampling from the incident wave, a portion having sufficient energy, is a power divider, and the measuring cell is placed in derivation relatively to the ultra-high frequency cable.

**9.** The device for hydrometric measurements, according to claim 1, wherein the external wall of the measuring cell is provided with slits directed along the wave propagation vector.

**10.** The device for hydrometric measurements, according to claim 1, wherein the external wall of the measuring cell is porous.

**11.** The device for hydrometric measurements, according to claims 1, 3 and 6 or to claims 1, 4 and 6, wherein the measuring cell includes a hollow cylinder-shaped cavity delimited by:

- an inner conducting cylindrical surface, also forming the shielding of the shrinked portion of the ultra-high frequency cable,
- an outer conducting cylindrical surface, electrically connected through its two ends to the shielding of both ultra-high frequency cable sections which surround it,
- the distal portion of this cavity consisting of a conducting washer putting both cylindrical surfaces and the downstream portion of the ultra-high frequency cable into contact over 360°,

this cavity being filled at its end turned towards the generator, with a dielectric identical with the one of the cable, and occupying all the space between both cylinders over a length of a few millimeters, and being filled in the remaining portion with the homogeneous dielectric material sample, for which the permittivity is a monotonous function of the hydrometry.

12. The device for hydrometric measurements, according to claims 1, 5 and 6, wherein the measuring cell includes a hollow cylinder-shaped cavity delimited by:

- an inner conducting cylindrical surface, with a diameter less than the smallest diameter of the dielectric surrounding both conductors,
- an outer conducting cylindrical surface,
- the distal portion of this cavity consisting of a conducting washer putting both cylindrical surfaces into contact over 360°,

this cavity being filled at its end turned towards generator, with a dielectric identical with the one of the cable and occupying all the space between both cylinders over a length of a few millimeters, and being filled in the remaining portion with the homogenous dielectric material sample for which permittivity is a monotonous function of the hydrometry.

13. The device for hydrometric measurements, according to any of claims 1 to 12, **characterized in that** one or more distal measuring cells sample a larger proportion of the incident microwave than the measuring cells closest to the source.

14. The device for hydrometric measurements, according to any of claims 1 to 12, **characterized in that** the dielectric of the ultra-high frequency cable and of the measuring cell have a continuous structure.

15. The device for hydrometric measurements, according to any of claims 1 to 13, including a first generator of sine wave grains, a multiplexing device successively switching these wave trains to one end of several ultra-high frequency cables, a vector voltmeter (43) connected to each of these ultra-high frequency cables and electronic means with which the complex reflection coefficient may be calculated in the frequency domain, a Fourier transform may be performed in order to calculate the complex reflection coefficient in the time domain, and then the values of the real and imaginary parts of the permittivity may be determined in order to determine the measurement of humidity and temperature by correlation with tables of values experimentally established beforehand by means of another hydrometric measurement method.

16. The device for hydrometric measurements, according to claim 1, wherein the read-out means are located at the end of the ultra-high frequency cable, opposite to the one connected to the means for generating sine wave trains.

17. A hydrometric measurement assembly comprising at least one sensor according to any of claims 1 to 16, **characterized in that** the generator of sine wave trains and the electronic read-out means are formed with a network analyzer.

18. A hydrometric measurement assembly comprising at least one sensor according to any of claims 1 to 14, **characterized in that** the generator of sine wave trains is a frequency synthesizer, the electronic read-out means are formed with a vector voltmeter (43) associated with digital processing means.

**Patentansprüche**

1. Hydrometrische Höchstfrequenzmessvorrichtung, umfassend:

- elektronische Generatoreinrichtungen sinusförmiger Wellenzüge mit Frequenzen, die mehrere Werte zwischen einigen MHz und einigen GHz in arithmetischer Progression annehmen,
- mindestens ein Höchstfrequenzkabel, längs dem sich wenigstens zwei Messstationen (4) befinden, wobei jede Messstation einerseits eine Trenneinrichtung hat, fähig der eintreffenden Welle nur einen Teil zu entnehmen, der eine ausreichende Energie hat, so dass die Messzelle ein durch die elektronischen Leseeinrichtungen messbares Echo zurücksendet, und andererseits eine Messzelle (14) hat, gebildet durch ein Höchstfrequenzleitungs-Teilstück, dessen distales Ende mit einem Kurzschluss endet, wobei dieses Teilstück eine Außenwand hat, die porös ist oder Öffnungen aufweist und deren Dielektrikum im Wesentlichen durch eine Probe eines homogenen dielektrischen Materials gebildet wird, dessen Permittivität eine monotone Funktion der Hydrometrie in dem betreffenden Messbereich ist,
- elektronische Leseeinrichtungen, die aufgrund von Signalen, die das Höchstfrequenzkabel durchlaufen haben, ermöglichen, die Werte der reellen und imaginären Teile der Permittivität zu bestimmen, um das Maß der Feuchtigkeit und der Temperatur durch Korrelation mit Wertetabellen zu bestimmen, die vorher mit Hilfe einer anderen hydrometrischen Messmethode experimentell erstellt wurden.

**2.** Hydrometrische Messvorrichtung nach Anspruch 1, bei der die elektronischen Leseeinrichtungen Einrichtungen umfassen: um diese Signale zu digitalisieren, um sie einem Frequenzfiltern zu unterziehen, um den komplexen Reflexionskoeffizienten im Frequenzbereich zu berechnen, um eine Fourier-Transformierte zu ermitteln, um den komplexen Reflexionskoeffizienten im Zeitbereich zu berechnen und dann um die Werte der reellen und imaginären Teile der Permittivität zu bestimmen.

**3.** Hydrometrische Messvorrichtung nach einem der Ansprüche 1 oder 2, bei der die Leseeinrichtungen sich an demselben Ende des Höchstfrequenzkabels wie die Generatoreinrichtungen der sinusförmigen Wellenzüge befinden und mit diesem Höchstfrequenzkabel durch einen Richtungskoppler verbunden sind.

**4.** Hydrometrische Messvorrichtung nach einem der Ansprüche 1 bis 3, bei der das Höchstfrequenzkabel koaxial ist.

**5.** Hydrometrische Messvorrichtung nach einem der Ansprüche 1, 2 oder 3, bei der das Höchstfrequenzkabel bifilar abgeschirmt ist.

**6.** Hydrometrische Messvorrichtung nach einem der Ansprüche 1 bis 3, bei der das Höchstfrequenzkabel bifilar unabgeschirmt ist.

**7.** Hydrometrische Messvorrichtung nach einem der Ansprüche 1 bis 6, bei der die Messzelle mit dem Höchstfrequenzkabel koaxial ist und dieses auf Höhe dieser Zelle eine brüske Verengung aufweist.

**8.** Hydrometrische Messvorrichtung nach Anspruch 1 und einem der Ansprüche 3 bis 5, bei der die Einrichtung, die fähig ist, der eintreffenden Welle nur einen Teil mit einer ausreichenden Energie zu entnehmen, ein Leistungsteiler ist, und die Messzelle sich in einer Abzweigung in Bezug auf das Höchstfrequenzkabel befindet.

**9.** Hydrometrische Messvorrichtung nach Anspruch 1, bei der die Außenwand der Messzelle Schlitze aufweist, die entsprechend Ausbreitungsvektoren der Welle orientiert sind.

**10.** Hydrometrische Messvorrichtung nach Anspruch 1, bei der die Außenwand der Messzelle porös ist.

**11.** Hydrometrische Messvorrichtung nach den Ansprüchen 1, 3 und 6 oder den Ansprüchen 1, 4 und 6, bei der die Messzelle einen Hohlraum von hohler zylindrischer Form umfasst, abgegrenzt durch:

- eine leitfähige zylindrische Innenoberfläche, die auch die Abschirmung des eingeengten Teils des Höchstfrequenzkabels bildet,
- eine leitfähige zylindrische Außenoberfläche, elektrisch durch ihre beiden Enden mit der Abschirmung der beiden Höchstfrequenzkabelstücke, die sie umgeben, verbunden,
- den distalen Teil dieses Hohlraums, gebildet durch eine leitfähige Scheibe, die über 360° den Kontakt zwischen den beiden zylindrischen Oberflächen und dem flussabwärts befindlichen Teil des Höchstfrequenzkabels herstellt,

wobei dieser Hohlraum an seinem dem Generator zugewandten Ende mit dem gleichen Dielektrikum wie das Kabel gefüllt ist, das den ganzen Raum zwischen den beiden Zylindern über eine Länge von einigen Millimetern einnimmt, und in dem restlichen Teil mit der Probe aus homogenem dielektrischem Material gefüllt ist, dessen Permittivität eine monotone Funktion der Hydrometrie ist.

**12.** Hydrometrische Messvorrichtung nach den Ansprüchen 1, 5 und 6, bei der die Messzelle einen Hohlraum von hohler zylindrischer Form umfasst, abgegrenzt durch:

- eine leitfähige zylindrische Innenoberfläche, die auch die Abschirmung des eingeengten Teils des Höchstfrequenzkabels bildet,
- eine leitfähige zylindrische Außenoberfläche, elektrisch durch ihre beiden Enden mit der Abschirmung der beiden Höchstfrequenzkabelstücke, die sie umgeben, verbunden,
- den distalen Teil dieses Hohlraums, gebildet durch eine leitfähige Scheibe, die über 360° den Kontakt zwischen den beiden zylindrischen Oberflächen herstellt,

wobei dieser Hohlraum an seinem dem Generator zugewandten Ende mit dem gleichen Dielektrikum wie das Kabel gefüllt ist, das den ganzen Raum zwischen den beiden Zylindern über eine Länge von einigen Millimetern einnimmt, und in dem restlichen Teil mit der Probe aus homogenem dielektrischem Material gefüllt ist, dessen Permittivität eine monotone Funktion der Hydrometrie ist.

**13.** Hydrometrische Messvorrichtung nach einem der Ansprüche 1 bis 12, **dadurch gekennzeichnet, dass** eine (oder mehrere) distale Messzelle(n) der eintreffenden Höchstfrequenzwelle einen größeren Teil entnimmt (entnehmen) als die am nächsten bei der Quelle befindlichen Zellen.

**14.** Hydrometrische Messvorrichtung nach einem der Ansprüche 1 bis 12, **dadurch gekennzeichnet, dass** die Dielektrika des Höchstfrequenzkabels und der Messzelle eine kontinuierliche Struktur haben.

15. Hydrometrische Messvorrichtung nach einem der Ansprüche 1 bis 13, mit einem selben Generator sinusförmiger Wellenzüge, einer Multiplexeinrichtung, die diese Wellenzüge sukzessiv auf ein Ende von mehreren Höchstfrequenzkabeln schaltet, einem mit jedem dieser Höchstfrequenzkabel verbundenen Vektorvoltmeter (43) und den elektronischen Einrichtungen, die ermöglichen, den komplexen Reflexionskoeffizienten im Frequenzbereich zu berechnen, eine Fourier-Transformierte zu ermitteln, um den komplexen Reflexionskoeffizienten im Zeitbereich zu berechnen und dann die Werte der reellen und imaginären Teile der Permittivität zu bestimmen, um das Maß der Feuchtigkeit und der Temperatur durch Korrelation mit Wertetabellen zu bestimmen, die vorher mit Hilfe einer anderen hydrometrischen Messmethode experimentell erstellt wurden.

16. Hydrometrische Messvorrichtung nach Anspruch 1, bei der sich die Leseeinrichtungen an dem Ende des Höchstfrequenzkabels befinden, das demjenigen entgegengesetzt ist, das mit den Generatoreinrichtungen sinusförmiger Wellenzüge verbunden ist.

17. Hydrometrisches Messsystem mit wenigstens einem Sensor nach einem der Ansprüche 1 bis 16, **dadurch gekennzeichnet, dass** der Generator sinusförmiger Wellenzüge und die elektronischen Leseeinrichtungen durch einen Netzanalysator gebildet werden.

18. Hydrometrisches Messsystem mit wenigstens einem Sensor nach einem der Ansprüche 1 bis 14, **dadurch gekennzeichnet, dass** der Generator sinusförmiger Wellenzüge ein Frequenzsynthesator ist und die elektronischen Leseeinrichtungen durch ein Vektorvoltmeter (43) gebildet werden, das mit digitalen Verarbeitungseinrichtungen verbunden ist.

FIG. 1

FIG. 2

# FIG. 3

# FIG. 4

FIG. 5

EP 1 685 389 B1